# EUROPEAN PATENT SPECIFICATION

(11) **EP 1 748 066 B1**
(45) Date of publication and mention of the grant of the patent: **31.12.2008**
(21) Application number: 05016549.7
(22) Date of filing: 29.07.2005
(51) Int. Cl.: C12N 5/08, A61K 35/32, A61P 19/00

(54) **Method for isolating stem cells from a pad-like tissue of teeth**
Verfahren zur Isolierung von Stammzellen aus ein kissenformiges Gewebe der Zähne
Méthode pour isoler des cellules souches d'un tissu dentaire en coussinet

(43) Date of publication of application: 31.01.2007
(73) Proprietor: Stiftung CAESAR, 53175 Bonn (DE); Siemonsmeier, Jürgen, Dr., 53127 Bonn (DE); Rheinische Friedrich-Wilhelms-Universität Bonn, 53113 Bonn (DE)
(72) Inventor: Siemonsmeier, Jürgen, Dr.Dr., D-53127 Bonn (DE); Degistirici, Özer, Dr., 53177 Bonn (DE); Thie, Michael, Dr., 53175 Bonn (DE); Götz, Werner, Prof.Dr., 53125 Bonn (DE)
(74) Representative: Schrooten, Rolf

(56) References cited:
- WO-A-02/07679
- WO-A-03/066840
- WO-A-20/04094588
- MORSCZECK C ET AL: "Isolation of precursor cells (PCs) from human dental follicle of wisdom teeth" MATRIX BIOLOGY, vol. 24, no. 2, April 2005 (2005-04), pages 155-165, XP004884874 ISSN: 0945-053X
- GRONTHOS S ET AL: "Postnatal human dental pulp stem cells (DPSCs) in vitro and in vivo" PROCEEDINGS OF THE NATIONAL ACADEMY OF SCIENCES OF USA, vol. 97, no. 25, 5 December 2000 (2000-12-05), pages 13625-13630, XP002201506 ISSN: 0027-8424
- HARADA H ET AL: "Localization of putative stem cells in dental epithelium and their association with Notch and FGF signaling" THE JOURNAL OF CELL BIOLOGY, vol. 147, no. 1, 4 October 1999 (1999-10-04), pages 105-120, XP002237265 ISSN: 0021-9525

## Description

### Field of the Invention

The invention concerns a method for isolating non-embryonic stem cells from a tissue that is located in immediate vicinity of immature, developing teeth or wisdom teeth. The invention further relates to non-embryonic stem cells derived from said tissue.

### Background of the Invention

Stem cells are self-renewing cells that divide to give rise to a cell with an identical developmental potential and/or with a more restricted developmental potential, i.e. stem cells can divide asymmetrically to yield a stem cell and a more specialised cell which has lost some developmental potential. A stem cell has the ability to divide for indefinite periods, at least through many cycles, and often throughout the whole life of the organism. Given specific signals in the development of the organism, stem cells can differentiate into many different cell types that make up the organism. That is, stem cells have the potential to develop into mature cells that have characteristic shapes and specialised functions, such as bone cells, muscle cells, skin cells, or nerve cells. Unipotent stem cells produce a single type of differentiated cell, whereas pluripotent stem cells produce most cell types of the body.

Pluripotent stem cells derived from the inner cell mass of blastocysts (Embryonic Stem cells = ES cells) can give rise to cells that can form a wide range of cell types, such as the mesoderm, endoderm, and ectoderm of an embryo, and much attention has therefore been focused in the past on ES cells. However, ethical concerns have been raised about the use of embryonic stem cells. Thus, non-embryonic stem cells, i.e. somatic stem cells (Adult Stem cells = AS cells), are now of increasing importance for developing innovative therapeutic strategies.

An adult (somatic) stem cell (AS cell) is an undifferentiated cell that is situated in a differentiated tissue but can renew itself and become specialised to yield all of the specialised cell types of the tissue from which it originated, and possibly other specialised cells. For example, stem cells harvested from bone marrow have been used in therapy in cases of leukemia, lymphomas, and other life-threatening diseases. Recent studies have demonstrated that mesenchymal stem cells derived from bone marrow may be able to generate both skeletal muscle and neurons. Thus, there is evidence that AS cells derived from a specific type of tissue can generate mature, fully functional cells of another type, and that after exposure to a new environment, they may be able to populate other tissues and possibly differentiate into other cell types.

WO 01/60981 A1 discloses a method for producing tooth progenitor cells using cultured embryonic stem cells (ES cells). Production of the progenitor cells is here employed by inducing differentiation of the ES cells with oral epithelial cells. However, this approach uses embryos as a source for stem cells, so that beside ethical concerns also availability of the source material is limited.

WO 02/07676 A2 describes a method for regenerating human dentin and pulp using cultured adult stem cells (AS cells) derived from dental pulp. However, since non-embryonic stem cells from pulp are already in an advanced state of development they are not multipotent but merely differentiate into the specialised cell type of the tissue from which they were isolated. Moreover, obtaining the cells is very difficult because the tooth has to be cracked to make the pulp accessible.

WO 03/066840 A2 discloses a method for isolating non-embryonic stem cells derived from the dental follicle (dental sac) of tooth germs. The resulting stem cells are stated to be pluripotent and capable of differentiation into specialised cells of endodermal, ectodermal and mesodermal lineages. Although these stem cells may be a very promising parent material for several therapeutic approaches, isolation of the cells is difficult since only small amounts of follicle cells reside around a tooth. Moreover, this tissue is partially destroyed when the tooth is removed by usual surgical methods since parts of the dental follicle remain within the jaw attached to the tissue of the alveolar fundus surrounding the tooth.

### Summary of the Invention

It is the object of the invention to provide a method for isolating non-embryonic stem cells, which can be easily accomplished and yields a considerable amount of cells, and non-embryonic stem cells that are at least multipotent and can be easily obtained in a considerable amount.

The object is met by a method for isolating non-embryonic stem cells comprising the steps of
- identifying a pad-like tissue located in immediate vicinity of the apical side of a surgically removed immature, developing tooth or wisdom tooth underneath the dental papilla, wherein said tooth or wisdom tooth is surgically removed in a development stage between the appearance of the bony alveolar fundus and the end of the formation of the root of said tooth or wisdom tooth,
- separating said pad-like tissue from said dental papilla and/or said root, and
- singularising cells by disintegrating said pad-like tissue.

The method according to the invention utilises a living soft tissue residing undemeath the dental papilla in immediate vicinity of the apical side of a developing tooth, which is clearly distinguished from other tooth tissue, such as dental papilla or follicle (dental sac including operculum). In the stage of development between the appearance of the bony alveolar fundus and the end of the formation of the root, no dental follicle tissue is located at the apical side of the immature tooth but merely lateral follicle can be observed. That is, the follicular cells are either dissipated to form cementum, periodontal ligament and/or alveolar bone or migrated towards the crown. However, when a non-erupted, immature tooth is surgically removed a pad-like tissue is observed, which is attached to the dental papilla and/or the root at the apical side of the developing tooth. This valuable tissue is a white, pad-like soft tissue structure, which approximately has the size of a lentil or a bean, depending on the developmental stage. The pad-like tissue can only be detected in a defined, specific developmental stage in an early phase of root formation. That is, identifying and separating the pad-like tissue is only possible from the appearance of the bony alveolar fundus to the end of the formation of the root of the tooth.

In usual dental practice, merely the lateral and occlusal parts of the tissue surrounding the developing tooth, i.e. the remaining parts of the dental follicle, are admitted to histological analysis, whereas the apical soft tissue structure is discarded together with the immature tooth. That is, the potential of the soft tissue residing at the apical side of an immature, developing tooth was unknown by now and thus has been disregarded so far. However, once isolated using the method according to the invention the apical pad-like tissue shows its enormous potential as it surprisingly comprises at least multipotent stem cells which can differentiate as ectomesenchymal stem cells (mesenchymal cells derived from the ectodermal neural crest).

Identification and isolation of the pad-like tissue is easy and can be accomplished using appropriate standard surgical equipment. Since the pad-like tissue is a by-product of surgically removed non-erupted teeth it is easily available without limitation. Additionally, since the pad-like tissue has the size of a lentil or a bean, the method according to the invention yields a quantity of useful singularised cells that is sufficient for transplantation or other medical purposes. Moreover, the resulting stem cells are at least multipotent and can be used for producing functional cells and tissues of several types, even types that are different from tooth tissue. That is, the multipotent stem cells according to the invention can give rise to differentiated progeny and daughter cells that develop into all varieties of neural crest derivations.

In an advantageous embodiment of the invention, the pad-like tissue is separated from the dental papilla and/or the root by dissection, in particular by cutting along the macroscopically visible border between the dental papilla and the pad-like tissue. Thus, the pad-like tissue can be surgically harvested without using any sophisticated technical equipment. The border between the dental papilla and the pad-like tissue can be macroscopically detected and hence precise separation of the pad-like tissue can be accomplished. However, in some cases it may be difficult to exactly identify this border if the soft tissue attached to the surgically removed tooth has been affected, e.g. during transport. But even in those cases it is still possible at least to enrich the ectomesenchymal stem cells by surgically harvesting the soft tissue underneath the fictitious line between the roots of the tooth.

Subsequently, the pad-like tissue is disintegrated by enzymatic digestion, e.g. the cells are dissociated using collagenase and/or dispase in serum-free cell culture medium. The resulting singularised cells may be cultured and expanded in an appropriate medium, e.g. a standard cell culture medium containing fetal calf serum (FCS). The stem cells are highly proliferative and may be cultured as adhering cells over several cycles. The adhering cells are initially morphologically diverse, i.e. they comprise flat, disc-like cells, spindle-shaped cells, and spherical cells. In the end, fibroblast-like cells dominate the culture forming a confluent multilayer.

In a preferred embodiment of the invention, the tooth or wisdom tooth is a mammal tooth, preferably a human tooth. If the origin of the stem cells is human tooth tissue, the method according to the invention is the first step of promising cell therapies for humans, e.g. in respect of threatening disorders like cancer or degenerative diseases.

The singularised cells may be transfected with a nucleic acid, in particular DNA, preferably comprising at least one gene of interest and/or at least one marker sequence. Genetically engineering the resulting stem cells to express a gene of interest may be a beneficial approach for certain applications, for example, if a special therapy using transplanted stem cells has to be supported by a specific protein or peptide.

In a special embodiment of the invention, the singularised cells are stimulated to differentiate into bone-forming cells. In this case, osteogenic stimulation can be achieved by supplying the cells with 10⁻⁷ M dexamethasone, 50 µg/ml ascorbic acid 2-phosphate, and 10 mM β-glycerolphosphate. Bone-forming matrix can be detected, for example, by staining with alizarine red. As a result, the method according to the invention may be employed to achieve stem cells for regeneration or replacement of cranial bony tissue or parts of the skeletal system.

The object is also met by a non-embryonic stem cell isolated from a pad-like tissue located in immediate vicinity of the apical side of an immature, developing tooth or wisdom tooth underneath the dental papilla, wherein said tooth or wisdom tooth is in a stage of development between the appearance of the bony alveolar fundus and the end of the formation of the root of said tooth or wisdom tooth. The present invention further comprises a non-embryonic stem cell, which is isolated by the method according to the invention. Such stem cells are at least multipotent and therefore able to produce functional cells and tissues of several developmental paths, even tissues that are different from tooth tissues. Since isolation of the pad-like tissue can be easily accomplished using appropriate standard equipment and the pad-like tissue has the size of a lentil or a bean, the non-embryonic stem cells according to the invention can be obtained in a considerable amount that is sufficient for transplantation or other medical purposes. The multipotent stem cells according to the invention can give rise to differentiated progeny and daughter cells that develop into all varieties of neural crest derivations. Hence, the stem cells according to the invention have the potential to generate lineages of neural crest-derivated cells and their differentiated cells. Since the ectomesenchymal stem cells according to the invention are derived from a tissue in an early development stage, e.g. in contrast to stem cells derived from pulp, they have an enormous potential as progenitor cells for many scientific and medical applications. Thus, the stem cells according to the invention differ from tooth-derived stem cells known from the prior art.

In a preferred embodiment of the invention, the stem cell according to the invention is a mammalian cell, preferably a human cell, and hence a promising source material for successful cell therapy for humans or other mammals.
In a particularly preferred embodiment of the invention, the non-embryonic stem cell is transfected with a nucleic acid, in particular DNA, preferably comprising at least one gene of interest and/or at least one marker sequence. That is, the present invention also relates to genetically engineered stem cells expressing at least one gene of interest.

The present invention also encompasses lineage-committed stem cells, either stem cell clones isolated by the method according to the invention or genetically engineered stem cells, which are at least multipotent and capable of producing cell lines that are committed to neural crest-derived lineages. In particular, the invention relates to isolation of at least multipotent somatic stem cells, preferably ectomesenchymal stem cells, and such isolated stem cells, respectively.

Any cell culture or cell structure comprising at least one non-embryonic stem cell according to the invention and pharmaceutical compositions comprising at least one non-embryonic stem cell according to the invention are subject to the invention as well. The cell cultures or cell structures which include at least one non-embryonic stem cell according to the invention can give rise to functional tissues or organs of several types. The pharmaceutical compositions according to the invention are based on the at least multipotent stem cells according to the invention and allow for treating various diseases or disorders resulting from cellular deficiencies. Possible therapies include repair or replacement of cells, tissues or organs by administration and/or transplantation of stem cells, cell structures or cell cultures according to the invention, or tissues or molecules derived therefrom. A cell therapy can be accomplished by administering to a patient, preferably a mammal, in particular a human being, an amount of the stem cells according to the invention, which is sufficient to cause a therapeutic effect. Besides usual auxiliary and/or carrier substances, the pharmaceutical compositions according to the invention may further comprise proliferation factors and/or lineage-commitment factors affecting the stem cells according to the invention.

The stem cells isolated by the method according to the invention or the stem cell according to the invention or the above-mentioned cell culture, cell structure, or pharmaceutical compositions each can be used for repairing or replacing damaged or destroyed tissue in vivo, for obtaining differentiated craniofacial cells or tissue including cells or tissue of the stromatognathic system, for obtaining differentiated neurons or nerve tissue, and/or for replacing or repairing ectomesenchymal tissue. Accordingly, as the stem cells are at least multipotent a wide range of applications can be covered based on the present invention.

The invention is described below in detail with reference to the drawings.

### In the figures:

- Figure 1: represents a CT-Scan (CT = computed tomography) of a developing upper tooth, a) axial view, b) frontal view, c) sagittal view;
- Figure 2: represents a sagittal section through an unerupted developing human tooth;
- Figure 3: is a photograph of two surgically removed developing wisdom teeth (3^{rd} molar);
- Figure 4: shows a tissue section through the apical soft tissue of the wisdom tooth according to Figure 3 showing histological details of the apical dental region, H.E. staining;
- Figure 5: represents a micrograph of isolated stem cells according to the invention;
- Figure 6: shows flow cytometric analyses of stem cells according to the invention, the cells being analysed for surface markers with CD 90, CD 73, CD 49e, CD 45, CD 31 and CD 13 specific antibodies, FITC = fluoresceinisofhiocyanate-coupled antibody, PE = phycoerythrin-coupled antibody, APC = Allophycocyanin-coupled antibody;
- Figure 7: shows a periostin-specific staining of a tissue section through the apical soft tissue of a removed developing tooth;
- Figure 8: represents a micrograph of stem cells according to the invention after osteogenic stimulation, the sample being stained with alizarin red.

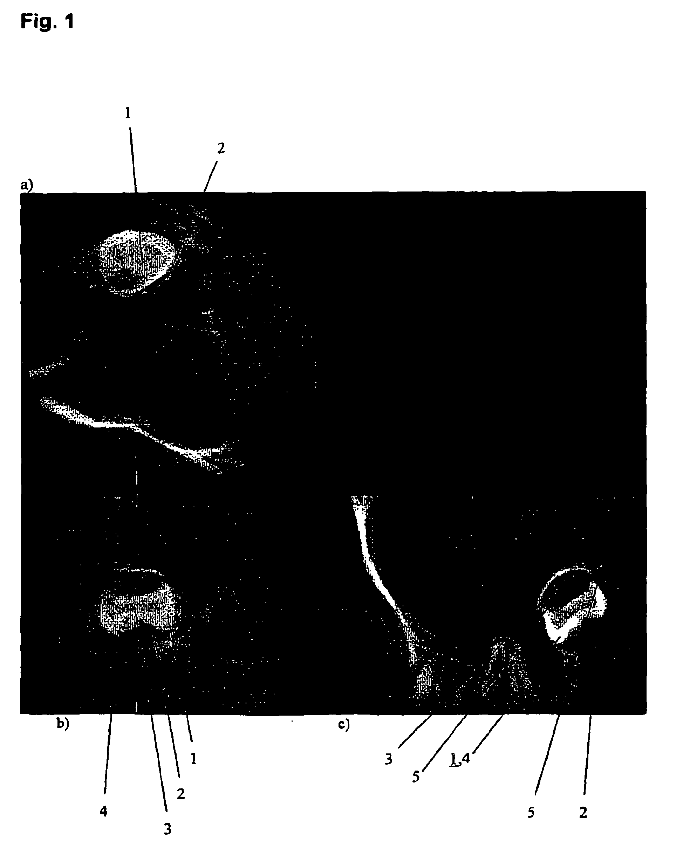

**Figure 1** represents a CT-Scan (CT = computed tomography) of a developing, unerupted upper tooth. The axial view a) shows the occlusal side of a developing tooth 1. That is, the coronary part including mucosa above the tooth 1 and operculum. The circumferential portion of the dental follicle 2 (lateral follicle) can also be observed in this representation. In frontal view b) and sagittal view c) of the tooth 1 a pad-like tissue 3 at the apical side (upper side in this representations) of the tooth 1 can be observed. The occlusal portion of the dental follicle 2 is located underneath the crown 4 of the tooth 1 diametrically opposed to the pad-like tissue 3. The pad-like tissue 3 according to the invention is situated above an imaginary line drawn between the roots 5 that are at the beginning of formation in this stage of tooth development.

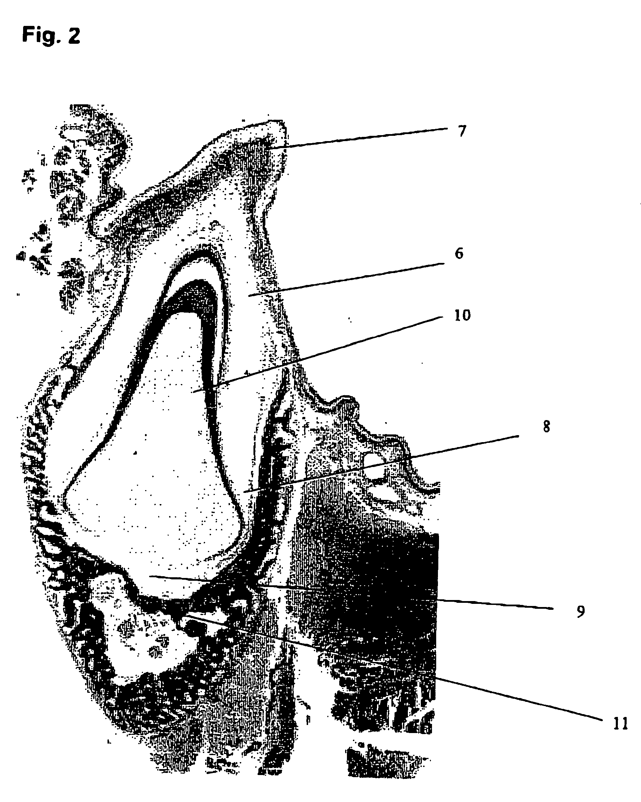

**Figure 2** represents a sagittal section through a developing unerupted human tooth 6. At the occlusal side of the tooth 6 the operculum 7 including mucosa can be observed, while the dental follicle 8 surrounds the lateral sides of the tooth 6. The pad-like tissue 9 is located at the apical side of the tooth 6 between the dental papilla 10 and the alveolar bone 11. Thus, at its apical side the pad-like tissue 9 contacts the occlusal surface of the alveolar bone 11.

According to the invention immature phase I or phase II teeth are used as source material. It is an advantage of this material that it is easily available in a significant number, particularly with children or adolescent persons. The developmental stage from the end of the formation of the crown 4 to the beginning of the formation of the root 5 is referred to as Phase I (see **Figure 1**), while Phase II is the root forming stage. In order to avoid contamination with oral or subgin-gival bacteria the oral cavity has to be disinfected prior to removal of the tooth, for example by rinsing with "Betaisodona" or "Chlorhexamed" solution. After typical dissection and osteotomy the bony cover above the alveolar compartment is removed. Subsequently, the tooth is carefully removed and further treated with care.

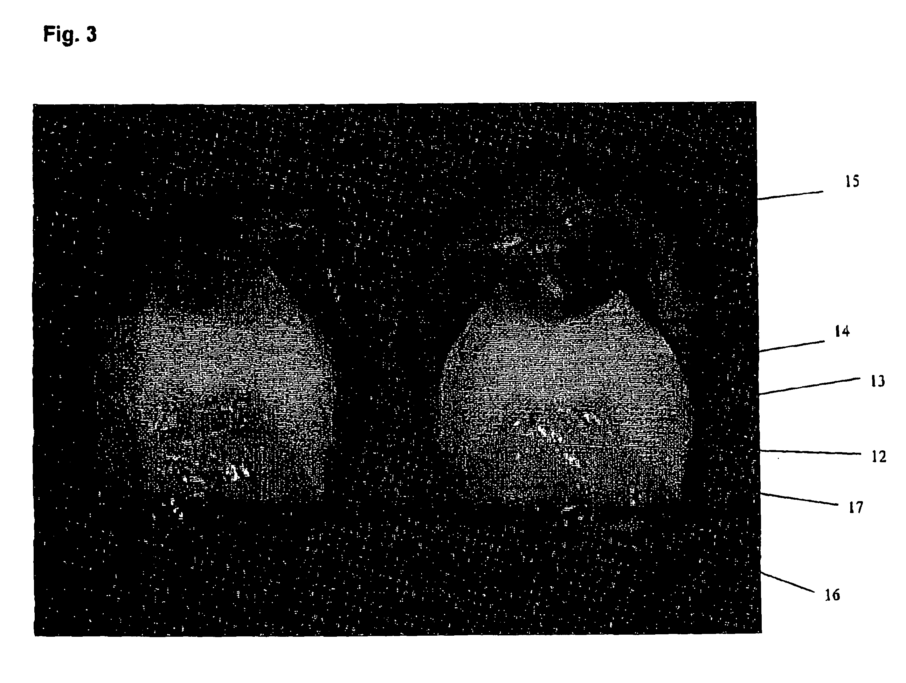

**Figure 3** is a photograph of surgically removed phase I wisdom teeth (3^{rd} molar). The tooth encompasses the dental papilla 12, the dental follicle 13, the enamel organ and its residual parts, respectively, as well as hard tissue of the crown 14 (enamel, dentine, and possibly cement). Under aseptic conditions, the coronary portion, i.e. mucosa above the tooth and operculum 15, is removed and discarded or used for histological analysis. The pad-like tissue 16 located at the apical side of the tooth underneath the dental papilla 12 is precisely separated from the dental papilla 12 and, if necessary, from the developing root 17, e.g. by cutting along the macroscopically visible border between the dental papilla 12 and the pad-like tissue 16 with a scalpel. The pad-like tissue 16 is a white, jelly-like tissue which approximately has the size of a lentil or a bean, depending on the stage of development of the developing tooth. This tissue comprises undifferentiated ectomesenchymal (neuroectodermal) stem cells.

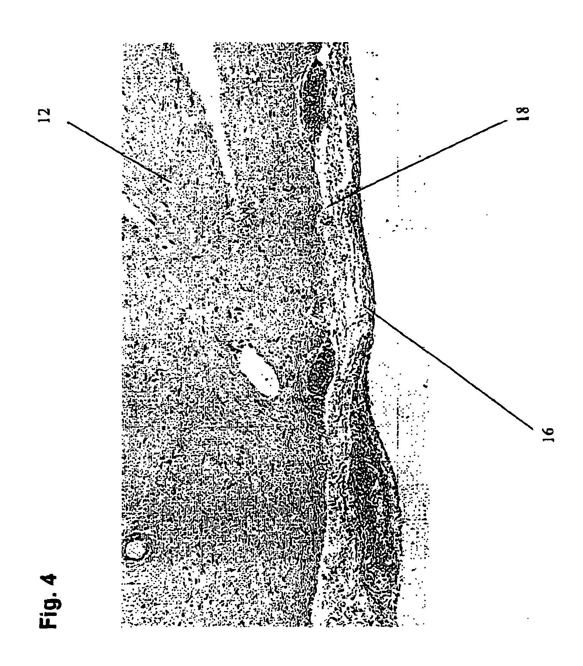

**Figure 4** shows a tissue section through the apical soft tissue of the wisdom tooth according to Figure 3 showing histological details of the apical dental region by H.E. staining. This histological analysis shows that the pad-like tissue 16 is a connective tissue with a low level of collagen fibres. White no adipose tissue can be observed in the pad-like tissue 16, it shows capillary blood vessels and nerve fibres. Histologically, condensed connective tissue or loose, vascularized connective tissue can be observed in the border region 18 between the dental papilla 12 and the occlusal side of the pad-like tissue 16. This border region 18 approximately corresponds to the location of the epithelial diaphragm which is a process of Hertwig's epithelial borderline that turns from lateral to central and relates to root formation with multi-root teeth in later stages of development. In contrast to the dental papilla 12, which has a high cell density and includes numerous blood vessels and nerve fibres, the apical pad-like tissue 16 is characterised by a low cell density, a low matrix level and a lower level of blood vessels and nerve fibres. The cells of the pad-like tissue 16 are flat and spindle-shaped and aligned parallel to the surface of the tissue in the apical border zone. In contrast, the cells of the dental papilla 12 are star-shaped cells having spherical nuclei, which are randomly distributed in the tissue.

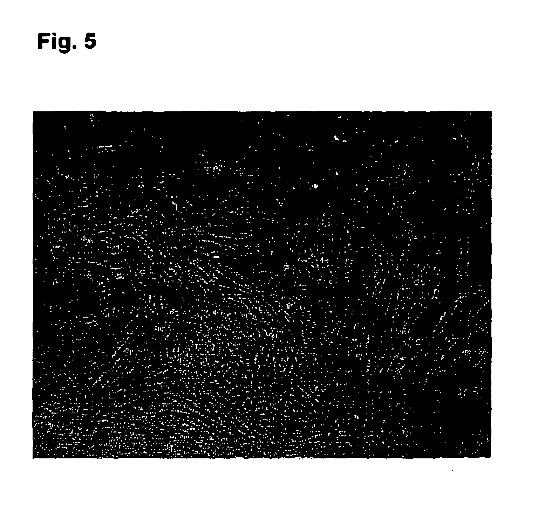

**Figure 5** represents a micrograph of isolated stem cells according to the invention. To this end, separated pad-like tissue was digested with collagenase / dispase in serum-free medium for 1 h at 37°C. After disintegrating the tissue, the singularised cells were placed in a T25 cell culture flask in a medium comprising DMEM low glucose (1 g/l), 10 % fetal calf serum (FCS) and 1 % penicillin / streptomcin / glutamine, and cultured at 37°C in a humidified atmosphere of 5% CO₂. After 7 to 14 days of incubation, formation of single colonies of fibroblastoid cells occurred (**Figure 5**). When the cells reached approximately 50 to 60 % confluency they were passaged to a new culture flask (5,000 cells per cm²) and cultured further. The cells of the pad-like tissue according to the invention can be easily cultured as adhering cells and are highly proliferative. The adhering cells can be classified into morphologically different cell types, i.e. flat, disc-like cells, spindle-shaped cells and spherical cells. These cells show a heterogeneous appearance which changes in the course of time until, in the end, fibroblast-like cells dominate the culture forming a confluent multilayer. A cell population can be described by Vim⁺ / nestin⁺ / PanZyto⁻ classification, which forms single nodule-like structures in a monolayer culture. Using ultrastructure analysis it can be demonstrated that these nodule-like structures encompass multilayered cells (fibroblasts / fibrocytes) which produce a loose extracellular matrix (collagen) and additionally further proteins (proteoglycans).

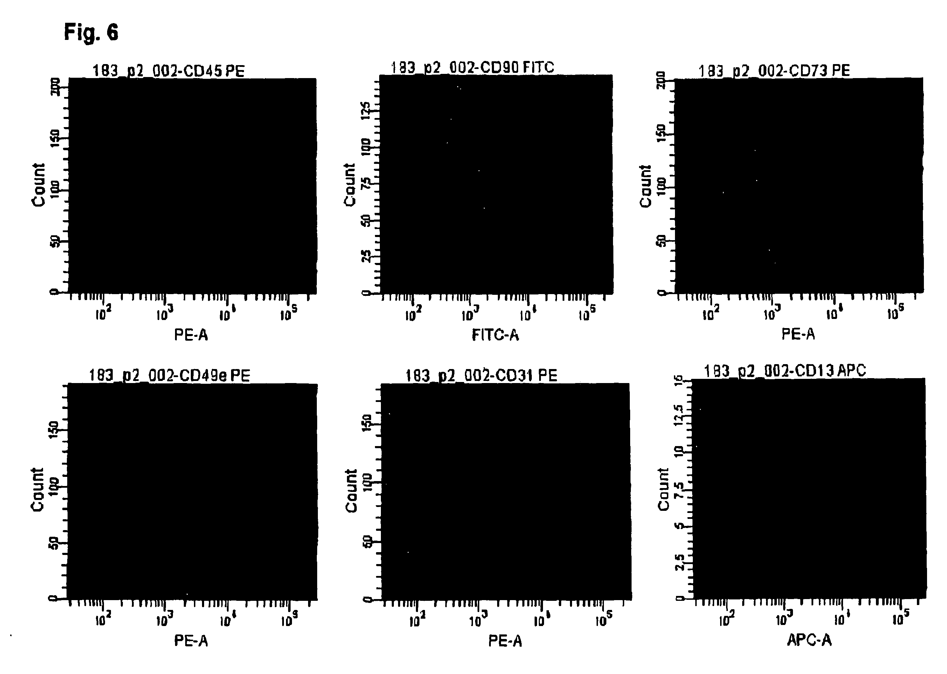

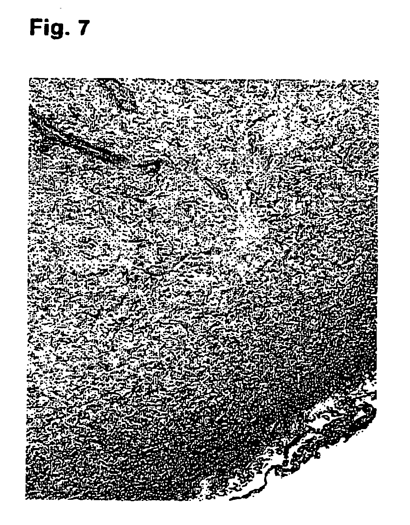

**Figure 6** shows flow cytometric analyses of stem cells according to the invention, wherein the cells are analysed for surface markers with CD 90, CD 73, CD 49e, CD 45, CD 31 and CD 13 specific antibodies. Approximately 60 surgically removed, non-pathological teeth were analysed histochemically using PAS and alcian blue staining and immunohistochemically using different antibodies. In these assays, marker for mesenchymal, endothelial and hematopoietic stem and progenitor cells, e.g. CD-90, CD-73, CD-49e, CD-45, CD-31, and CD-13 **(****Figure 6****),** intermediate Filaments (e.g. vimentin, keratins), as well as marker for differentiation of fibroblastic, osteoblastic and cementoblastic cells, e.g. BSP, vstoepontin, collagen, fibronectin, periostin, CAP (**Figure 7**), could be detected. Preliminary results indicate that the pad-like tissue contains more proteoglycans than dental pulp tissue and that discrimination from the neighbouring tissues may be possible using markers CD 57, p75, CAP as well as osteopontin and BSP.

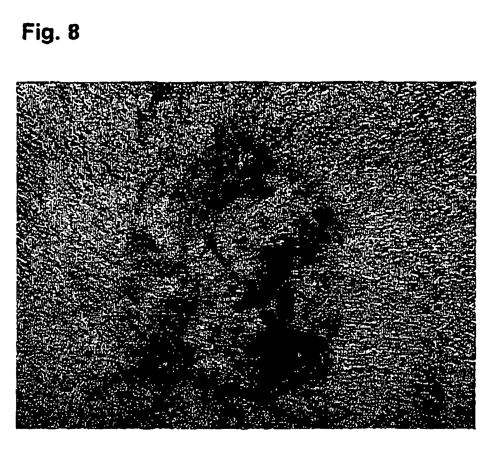

**Figure 8** represents a micrograph of stem cells according to the invention after osteogenic stimulation which can be achieved by supplying the cells with 10⁻⁷ M dexamethasone, 50 µg/ml ascorbic acid 2-phosphate and 10 mM β-glycerolphosphate. Bone-forming matrix can be detected by staining with alizarine red. The treated cells show significant calcium accumulation indicating the successful differentiation. This result demonstrates that the stem cells according to the invention can be stimulated for differentiation and that these cells are at least capable to give rise to osteoblasts.

### List of reference numbers:

- 1: Tooth
- 2: Dental follicle
- 3: Pad-like tissue
- 4: Crown
- 5: Root
- 6: Tooth
- 7: Operculum
- 8: Dental follicle
- 9: Pad-like tissue
- 10: Dental papilla
- 11: Alveolar bone
- 12: Dental papilla
- 13: Dental follicle
- 14: Crown
- 15: Operculum
- 16: Pad-like tissue
- 17: Root

## Claims

1. Ex vivo Method for isolating non-embryonic stem cells comprising the steps of
- identifying a pad-like tissue located underneath the dental papilla in immediate vicinity of the apical side of a surgically removed immature, developing tooth or wisdom tooth which is in a development stage between the appearance of the bony alveolar fundus and the end of the formation of the root of said tooth or wisdom tooth,
- separating said pad-like tissue from said dental papilla and/or said root, and
- singularising cells by disintegrating said pad-like tissue.

2. Method according to claim 1, **characterised in that** said pad-like tissue is separated from said dental papilla and/or said root by dissection, in particular by cutting along the macroscopically visible border between the dental papilla and the pad-like tissue.

3. Method according to claim 1 or 2, **characterised in that** said pad-like tissue is disintegrated by enzymatic digestion.

4. Method according to any one of claims 1 to 3, **characterised in that** said tooth or wisdom tooth is a human tooth.

5. Method according to any one of claims 1 to 4, **characterised in that** said singularised cells are transfected with a nucleic acid, in particular DNA, preferably comprising at least one gene of interest and/or at least one marker sequence.

6. Method according to any one of claims 1 to 5, **characterised in that** said singularised cells are stimulated to differentiate into bone-forming cells.

## Patentansprüche

1. Ex-vivo-Verfahren zum Isolieren von nichtembryonalen Stammzellen, das die folgenden Schritte umfaßt:
- Identifizieren eines kissenartigen Gewebes, das sich unter der Zahnpapille in unmittelbarer Nähe der apikalen Seite eines extrahierten unreifen, sich entwickelnden Zahns oder Weisheitszahns befindet, welcher sich in einer Entwicklungsphase zwischen dem Auftreten des knöchernen Alveolarfundus und dem Abschluß der Wurzelbildung dieses Zahns oder Weisheitszahns befindet,
- Abtrennen dieses kissenartigen Gewebes von der Zahnpapille und/oder der Wurzel, und
- Vereinzeln von Zellen durch Auflösen des kissenartigen Gewebes.

2. Verfahren nach Anspruch 1, **dadurch gekennzeichnet, daß** das kissenartige Gewebe von der Zahnpapille und/oder Wurzel durch Sezieren, insbesondere durch Schneiden entlang der makroskopisch sichtbaren Grenze zwischen der Zahnpapille und dem kissenartigen Gewebe, abgetrennt wird.

3. Verfahren nach Anspruch 1 oder 2, **dadurch gekennzeichnet, daß** das kissenartige Gewebe durch enzymatische Verdauung aufgelöst wird.

4. Verfahren nach einem der Ansprüche 1 bis 3, **dadurch gekennzeichnet, daß** es sich bei dem Zahn oder Weisheitszahn um einen menschlichen Zahn handelt.

5. Verfahren nach einem der Ansprüche 1 bis 4, **dadurch gekennzeichnet, daß** die vereinzelten Zellen mit einer Nukleinsäure, insbesondere DNA, die vorzugsweise mindestens ein interessierendes Gen und/oder mindestens eine Markersequenz enthält, transfiziert werden.

6. Verfahren nach einem der Ansprüche 1 bis 5, **dadurch gekennzeichnet, daß** die vereinzelten Zellen zur Differenzierung in knochenbildende Zellen stimuliert werden.

## Revendications

1. Méthode d'isolement ex vivo de cellules souches non embryonnaires comprenant les étapes consistant à :
- identifier un tissu de type coussinet situé en dessous de la papille dentaire au voisinage immédiat du côté apical d'une dent en cours de développement ou d'une dent de sagesse immature et extraite chirurgicalement, dont le stade de développement se situe entre l'apparition du fond de l'alvéole osseuse et la fin de la formation de la racine de ladite dent ou dent de sagesse,
- séparer ledit tissu de type coussinet de ladite papille dentaire et/ou de ladite racine, et
- dissocier les cellules en désintégrant ledit tissu de type coussinet.

2. Méthode selon la revendication 1, **caractérisée en ce que** ledit tissu de type coussinet est séparé de ladite papille dentaire et/ou de ladite racine par dissection, en particulier en coupant le long de la bordure macroscopiquement visible entre la papille dentaire et ledit tissu de type coussinet.

3. Méthode selon la revendication 1 ou 2, **caractérisée en ce que** ledit tissu de type coussinet est désintégré par digestion enzymatique.

4. Méthode selon l'une quelconque des revendications 1 à 3, **caractérisée en ce que** ladite dent ou dent de sagesse est une dent humaine.

5. Méthode selon l'une quelconque des revendications 1 à 4, **caractérisée en ce que** lesdites cellules dissociées sont transfectées avec un acide nucléique, en particulier de l'ADN, comprenant de préférence au moins un gène d'intérêt et/ou au moins une séquence marqueur.

6. Méthode selon l'une quelconque des revendications 1 à 5, **caractérisée en ce que** lesdites cellules dissociées sont stimulées de manière à se différencier en cellules formatrices d'os.
